# EUROPEAN PATENT SPECIFICATION

(11) **EP 4 070 855 B1**
(45) Date of publication and mention of the grant of the patent: **24.12.2025**
(21) Application number: 20882274.2
(22) Date of filing: 28.10.2020
(51) Int. Cl.: A61P 7/04, A61P 43/00, C07K 14/725, A61K 40/11, C12N 5/0783, C12N 15/62, C12N 9/40, A61K 48/00, A61K 40/31, A61K 40/42

(54) **B-CELL ANTIBODY RECEPTOR AND USE THEREOF**
B-ZELLEN-ANTIKÖRPERREZEPTOR UND VERWENDUNG DAVON
RÉCEPTEUR D'ANTICORPS DE LYMPOCYTE B ET SON UTILISATION

(30) Priority: 01.11.2019 JP 2019199624
(43) Date of publication of application: 12.10.2022
(73) Proprietor: Kyoto Prefectural Public University Corporation, Kyoto-shi, Kyoto 602-8566 (JP); Medinet Co., Ltd., Shinagawa-ku Tokyo 140-0012 (JP)
(72) Inventor: GOJO, Satoshi, Kyoto-shi, Kyoto 602-8566 (JP); KAMI, Daisuke, Kyoto-shi, Kyoto 602-8566 (JP); HOSHINO, Atsushi, Kyoto-shi, Kyoto 602-8566 (JP); MINAMI, Yoshito, Kyoto-shi, Kyoto 602-8566 (JP); SHIKUMA, Akira, Kyoto-shi, Kyoto 602-8566 (JP)
(74) Representative: J A Kemp LLP
(86) International application number: PCT/JP2020/040515
(87) International publication number: WO 2021/085504

(56) References cited:
- WO-A1-2018/140573
- ANONYMOUS MALTE ET AL: "Serum-Mediated Inhibition of Enzyme Replacement Therapy in Fabry Disease - PMC", JOURNAL OF THE AMERICAN SOCIETY OF NEPHROLOGY, vol. 27, no. 1, 30 April 2015 (2015-04-30), United States, pages 256 - 264, XP093285744, ISSN: 1046-6673, Retrieved from the Internet <URL:https://pmc.ncbi.nlm.nih.gov/articles/PMC4696578/> DOI: 10.1681/ASN.2014121226
- KALPANA PARVATHANENI; SCOTT DAVID: "Engineered FVIII- expressing cytotoxic T cells target and kill FVIII-specific B cells in vitro and in vivo", BLOOD ADVANCES, vol. 2, no. 18, 19 September 2018 (2018-09-19), pages 2332 - 2340, XP055648083
- MATSUMOTO, MASANORI: "III. Diagnostics and Medical, 4. Thrombotic Thrombocytopenic Purpura", THE JOURNAL OF THE JAPANESE SOCIETY OF INTERNAL MEDICINE, vol. 103, no. 7, 30 November 2013 (2013-11-30), JP , pages 1613 - 1621, XP009536611, ISSN: 0021-5384

## Description

### TECHNICAL FIELD

The present invention relates to a novel artificial receptor (B-cell antibody receptor; BAR), an immune cell, and a regenerative medicinal product (cell medicine).

### BACKGROUND ART

Various proteins exist in a body of an organism, and their functions maintain life activities. When a mutation occurs in a gene encoding a protein, the protein cannot be produced, or its activity is reduced, so that a disease may occur.

Hemophilia is an inherited disease due to quantitative and qualitative abnormalities of blood coagulation factors, and is classified into hemophilia A, hemophilia B, and the like, depending on the type of blood coagulation factor to be a cause. The symptoms vary depending on degree of activity of the blood coagulation factor, but if it becomes severe, intraarticular bleeding, intramuscular bleeding or the like may occur, resulting in impairment of joint function and movement. As a treatment of hemophilia, a replacement therapy for replenishing an insufficient blood coagulation factor from outside the body is performed.

Thrombotic thrombocytopenic purpura is a disease caused by dysfunction due to quantitative and qualitative abnormalities in ADAMTS13, cleavage enzyme of a von Willebrand factor (VWF), which may be congenital or acquired. This dysfunction induces excessive microthrombi, platelets are consumed and reduced by thrombogenesis, and organ failure associated with ischemic changes in various peripheral organs is caused. It has been reported that in the congenital case, it is caused by mutation of a gene encoding the protein, and in the acquired case, it is caused by autoantibody to the protein. As a treatment method, for congenital cases, the onset of the disease is prevented by infusing 10 ml/kg body weight of fresh frozen plasma every 2 weeks and replenishing ADAMTS 13 enzymes. For acquired cases, plasma exchange therapy is performed.

Lysosomal storage disease is a group of diseases in which a large amount of lipid or mucopolysaccharide is accumulated in lysosome due to genetic deficiency of an acidic degradation enzyme in the lysosome which is an intracellular organelle, and various symptoms such as swelling of liver and spleen, bone degeneration and central nerve disorder are exhibited, and currently, 60 types of diseases are included (Non Patent Literature 1).

The diseases classified as lysosomal storage disease are distinguished by the type of enzyme being deficient, and examples thereof include Fabry disease, Gaucher disease, Pompe disease, and mucopolysaccharidosis.

Fabry disease, which is one of lysosomal storage diseases, is a disease that occurs when glycoproteins GL-3 and Gb3 are not degraded due to genetic deficiency or activity reduction of an enzyme called α-galactosidase (α-GAL, hereinafter also simply referred to as "GLA"), and the undegraded glycoproteins accumulate in tissues and organs. As symptoms, neurological symptoms (pain), corneal opacities, skin symptoms, cardiac dysfunction, renal dysfunction and the like are observed. As a therapeutic method, formulated α-GAL is replenished by drip infusion to degrade glycoprotein, thereby ameliorating symptoms and suppressing progression (Non Patent Literature 2).

As a treatment of a disease caused by the fact that a protein function cannot be exerted due to a genetic mutation, such as hemophilia and lysosomal storage disease, replacement therapy is administered. However, in the case of a mutation having a deletion or a large deficiency of a protein of a coagulation factor or a responsible enzyme, there are not a few cases where a coagulation factor or a responsible enzyme to be replenished from outside the body is regarded as a foreign substance by immunity in the patient's body, and a neutralizing antibody against the coagulation factor or responsible enzyme replenished from outside the body is produced. In a case where an antibody is produced in the body of a patient, the enzyme cannot sufficiently exert its function, so that its therapeutic effect is attenuated. For example, in the treatment of Fabry disease, a case has been confirmed in which a neutralizing antibody against α-GAL is produced in the body of a patient, and the antibody binds to the administered α-GAL, thereby attenuating the therapeutic effect (Non Patent Literatures 3 and 4).

### CITATION LIST

### NON PATENT LITERATURE

NON PATENT LITERATURE 1: Japan Intractable Diseases Information Center, Section of lysosomal storage disease (Designated Intractable Disease 19): http://www.nanbyou.or.jp/entry/4061
NON PATENT LITERATURE 2: Sumitomo Dainippon Pharma Web page: https://healthcare.ds-pharma.jp/disease/fabry/index.html
NON PATENT LITERATURE 3: J Am Soc Nephrol 27: 256-264, 2016
NON PATENT LITERATURE 4: Thromb Haematol 2005; 94: 760-769

### SUMMARY OF INVENTION

### TECHNICAL PROBLEM

As described above, when an antibody against a coagulation factor or a responsible enzyme is produced in the body of a patient, a disease occurs, or a situation in which a therapeutic method cannot exert a sufficient effect occurs, and thus a technique for controlling functions of these antibodies is required.

### SOLUTION TO PROBLEM

In order to solve the above problems, the present inventors have extensively conducted studies, and resultantly found that the problems can be solved by eliminating B cells that produce a neutralizing antibody as a cause by an immune cell expressing a novel B-cell antibody receptor. That is, the present invention is as follows.
(1) A B-cell antibody receptor wherein an antibody binding domain a), a transmembrane domain b), an intracellular domain of a costimulatory factor c) and an intracellular domain of an activated receptor d) are combined, wherein the antibody binding domain a) comprises:
   - a full-length protein of α-GAL, and the base sequence encoding the full-length protein of α-GAL comprises a base sequence set forth in SEQ ID NO: 1, or a base sequence 95% or more identical to the base sequence set forth in SEQ ID NO: 1, having an identical function; or
   - an antibody recognition sequence region (D1 region) of α-GAL, and the base sequence encoding the antibody recognition sequence region (D1 region) of α-GAL comprises a base sequence set forth in SEQ ID NO: 2, or a base sequence 95% or more identical to the base sequence set forth in SEQ ID NO: 2, having an identical function.
(2) The B-cell antibody receptor according to (1), wherein a linker is inserted into a binding moiety of said a), b), c) or d).
(3) The B-cell antibody receptor according to any one of (1) to (2), wherein the transmembrane domain b) is selected from a group consisting of CD28, CD3ζ, CD4, and CD8α.
(4) The B-cell antibody receptor according to any one of (1) to (3), wherein the intracellular domain of a costimulatory factor c) is selected from a group consisting of OX40, 4-1BB, CD27, CD278, and CD28.
(5) The B-cell antibody receptor according to any one of (1) to (4), wherein the activated receptor d) is selected from a group consisting of TCR and NKG2D.
(6) A nucleic acid encoding the B-cell antibody receptor according to any one of (1) to (5)**.**
(7) A vector comprising the nucleic acid according to (6).
(8) An immune cell expressing a B-cell antibody receptor that has been transfected using the nucleic acid according to (6) or the vector according to (7).
(9) The immune cell according to (8), wherein the immune cell is an αβT cell, a CD8T cell, a CD4T cell, a γδT cell, or an NK cell.
(10) A regenerative medicinal product (cell medicine) comprising the immune cell according to (8) or (9) as an active ingredient.
(11) An immune cell expressing a B-cell antibody receptor according to any one of (1) to (5) for use in a method of treating and/or preventing lysosomal storage disease.
(12) The immune cell for use according to (11), wherein the lysosomal storage disease is Fabry disease.

### ADVANTAGEOUS EFFECTS OF INVENTION

The present invention provides an effective means for treatment of diseases caused by antibodies produced in the body of a patient, in particular, lysosomal storage disease.

### BRIEF DESCRIPTION OF DRAWINGS

FIG. 1 shows a conceptual diagram of treatment with a BAR-T cell according to the present invention.
FIG. 2 shows expression of BAR by BAR-T cells.
FIG. 3 shows expression of BAR by BAR-T cells obtained by transfecting BAR mRNA into human T cells by using MaxCyte mRNA transfer protocol.
FIG. 4 shows a method for creating hybridoma that produce anti-GLA monoclonal antibodies.
FIG. 5 shows generation of a sequence of scFv of anti-GLA antibody.
FIG. 6 shows an example of a plasmid vector into which a base sequence of the scFv of anti-GLA antibody (SEQ ID NO: 9) is inserted.
FIG. 7 shows an outline of an in vitro killing assay.
FIG. 8 shows results of in vitro killing assay (results of flow cytometry).
FIG. 9 shows a base sequence (SEQ ID NO: 1) encoding an amino acid sequence of a full-length protein (Full Length) of α-GAL, which is an example of the antibody binding domain a), and a lentiviral vector map of a BAR into which the base sequence is inserted.
FIG. 10 shows a base sequence (SEQ ID NO: 2) encoding an amino acid sequence of an antibody recognition sequence (D1 region) of α-GAL, which is an example of the antibody binding domain a), and a lentiviral vector map of a BAR into which the base sequence is inserted.
FIG. 11 shows results of a binding assay of GLA-mCherry for Transfectant in which the scFv of anti-GLA antibody is forcibly expressed.
FIG. 12 shows an example of a plasmid vector map for mRNA synthesis of full-length protein (FL) BAR of GLA.
FIG. 13 shows a base sequence (SEQ ID NO: 3) encoding an amino acid sequence of a spacer domain of ADAMTS13, which is an example of the antibody binding domain a), and an example of a plasmid vector map of a BAR into which the base sequence is inserted.
FIG. 14 shows a base sequence (SEQ ID NO: 3) encoding an amino acid sequence of a spacer domain of ADAMTS13 and a base sequence (SEQ ID NO: 4) encoding an amino acid sequence of a cysteine-rich domain of ADAMTS 13, which are an example of the antibody binding domain a), and an example of a plasmid vector map of a BAR into which the base sequences are inserted.
FIG. 15 shows a base sequence (SEQ ID NO: 1) encoding an amino acid sequence of a full-length protein (Full Length) of α-GAL, which is an example of the antibody binding domain a), and a retroviral vector map of a BAR into which the base sequence is inserted.
FIG. 16 shows a base sequence (SEQ ID NO: 2) encoding an amino acid sequence of an antibody recognition sequence (D1 region) of α-GAL, which is an example of the antibody binding domain a), and a retroviral vector map of a BAR into which the base sequence is inserted.
FIG. 17 shows an example of a plasmid vector map for mRNA synthesis of full-length protein (FL) BAR of GLA.
FIG. 18 shows a base sequence (SEQ ID NO: 3) encoding an amino acid sequence of a spacer domain of ADAMTS13, which is an example of the antibody binding domain a), and an example of a plasmid vector map of a BAR into which the base sequence is inserted.
FIG. 19 shows a base sequence (SEQ ID NO: 3) encoding an amino acid sequence of a spacer domain of ADAMTS13 and a base sequence (SEQ ID NO: 4) encoding an amino acid sequence of a cysteine-rich domain of ADAMTS 13, which are an example of the antibody binding domain a), and an example of a plasmid vector map of a BAR into which the base sequences are inserted.
FIG. 20 shows a base sequence (SEQ ID NO: 1) encoding an amino acid sequence of a full-length protein (Full Length) of α-GAL, which is an example of the antibody binding domain, and a retroviral vector map of a BAR into which the base sequence is inserted.
FIG. 21 shows a retroviral vector map into which a base sequence (SEQ ID NO: 36) of the scFv of anti-GLA antibody is inserted.
FIG. 22 shows a base sequence (SEQ ID NO: 37) encoding an amino acid sequence of ADAMTS 13, which is an example of the antibody binding domain, and a retroviral vector map of a BAR in which the base sequence is inserted.

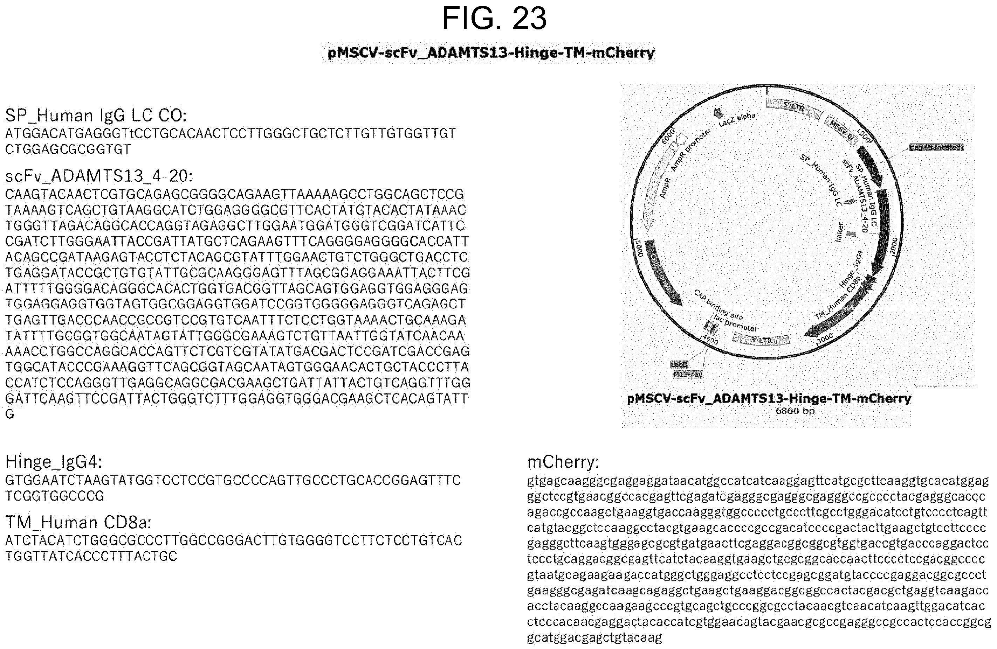

FIG. 23 shows a retroviral vector map into which a base sequence (SEQ ID NO: 41) of the scFv of anti-ADAMTS 13 antibody is inserted.
FIG. 24 shows results of a binding assay of hGLA-mCherry for Nalm6 in which the scFv of anti-GLA antibody is expressed.
FIG. 25 shows results of confirming expression of hGLA BAR using HA TAG.
FIG. 26 shows results of confirming expression of hGLA BAR using an anti-GLA antibody.
FIG. 27 shows results of a detection test for CD69 when BAR T cells (Jurkat-GLA-BAR2-EGFP: Effector) and target cells (NALM6-scFv-GLA-mCherry: Target) were mixed and cultured at mixing ratios of 1 : 0, 2 : 1, and 1 : 1 for 24 hours.
FIG. 28 shows results of confirming expression of ADAMTS13 BAR using HA TAG.
FIG. 29 shows results of confirming expression of ADAMTS13 BAR using HA TAG.

### DESCRIPTION OF EMBODIMENTS

The present invention provides a B-cell antibody receptor-immune cells for treating a disease that occurs when an antibody against a therapeutic enzyme or a coagulation factor or an endogenous protein replenished from outside body is produced in the body of a patient, and relates to immunotherapy using the cells. Here, the "B-cell antibody receptor-immune cell" (hereinafter, also simply referred to as "BAR-immune cell".) means an immune cell expressing a B-cell antibody receptor (BAR), and when the immune cell is a T cell, it is referred to as "B-cell antibody receptor-T cell" (hereinafter also simply referred to as "BAR-T cell").

### [1] B-Cell antibody receptor (BAR)

The BAR of the present invention contains an antibody binding domain a), a transmembrane domain b), an intracellular domain of a costimulatory factor c), and an intracellular domain of an activated receptor d). The BAR of the present invention has high specificity and cytotoxic activity against cells overexpressing antibodies to which the antibody binding domain a) binds.

The antibody binding domain a) of a BAR is not particularly limited as long as it is a substance to which an antibody causing disease specifically binds. If B cells that inhibit therapeutic effects of enzyme replacement therapy for lysosomal storage disease and the like are damaged and eliminated, for example, α-GAL in the case of Fabry disease, glucocerebrosidase in the case of Gaucher disease, α-glucosidase in the case of Pompe disease,α-L-iduronidase in the case of mucopolysaccharidosis, a full length protein of these proteins or a peptide containing an antibody recognition sequence or the like is selected as the antibody binding domain a). The amino acid sequences or base sequences of these proteins are publicly available and can be obtained by keyword search from the National Center for Biotechnology Information (NCBI) database.

This antibody binding domain binds to and interacts with an antibody causing disease, thereby damaging and eliminating B cells that produce such an antibody.

Further, if B cells that produce an antibody that inhibits therapeutic effects by administration of a blood coagulation factor like hemophilia are damaged and eliminated, for example, a full-length protein of blood coagulation factor VIII or blood coagulation factor IX, a peptide containing an antibody recognition sequence or the like is selected.

In addition, in the case of thrombotic thrombocytopenic purpura, examples thereof include a full-length protein of ADAMTS13, or a peptide containing an antibody recognition sequence, and the like.

In the BAR of the invention, the antibody binding domain includes a full-length protein (Full Length) of α-GAL or a peptide containing an antibody recognition sequence region (D1 region), and the amino acid sequence of the full-length protein (Full Length) of α-GAL and the base sequence encoding the same are shown in SEQ ID NO: 10 and SEQ ID NO: 1, and the amino acid sequence of the antibody recognition sequence region (D1 region) and the base sequence encoding the same are shown in SEQ ID NO: 11 and SEQ ID NO: 2.

The base sequence encoding the full-length protein (Full Length) of α-GAL or the antibody recognition sequence region (D1 region) may be a substantially homologous base sequence having the identical function. Here, the term "substantially homologous" encompasses two or more biomolecular sequences which are significantly similar to each other at the primary base sequence level. For example, in the context of two or more nucleic acid sequences, the "substantially homologous" means at least 95% identical, more preferably at least 97% identical, more preferably at least about 98% identical, and more preferably at least 99% identical.

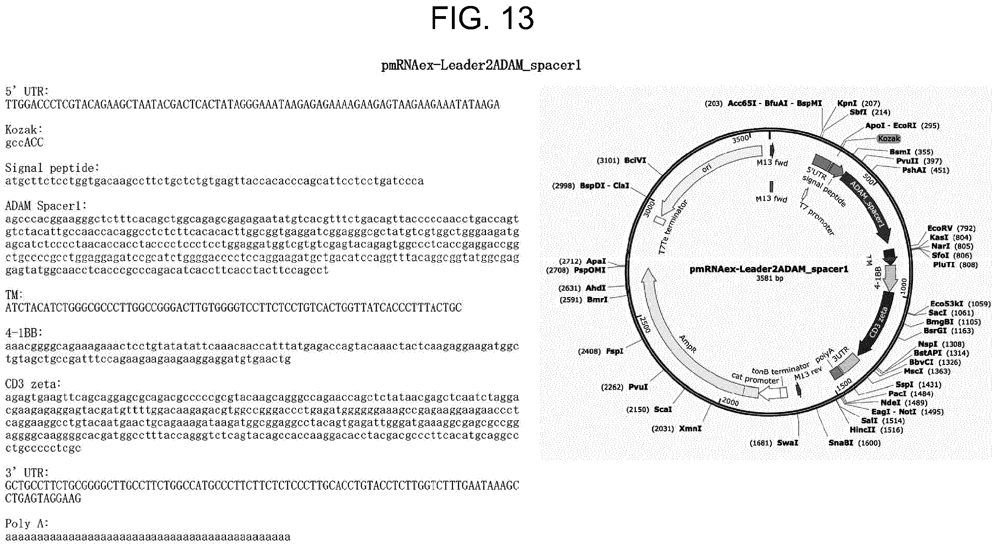

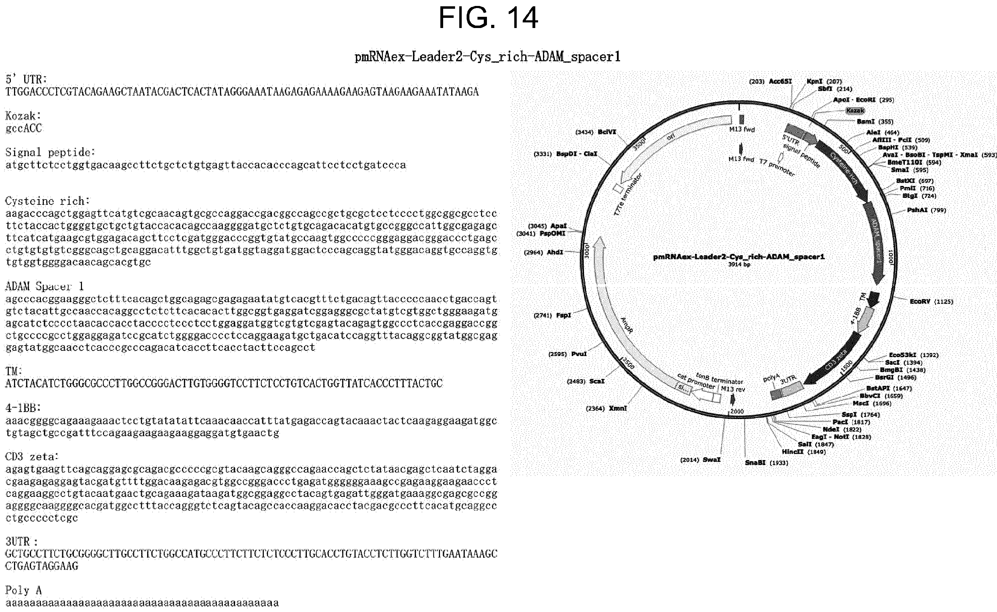

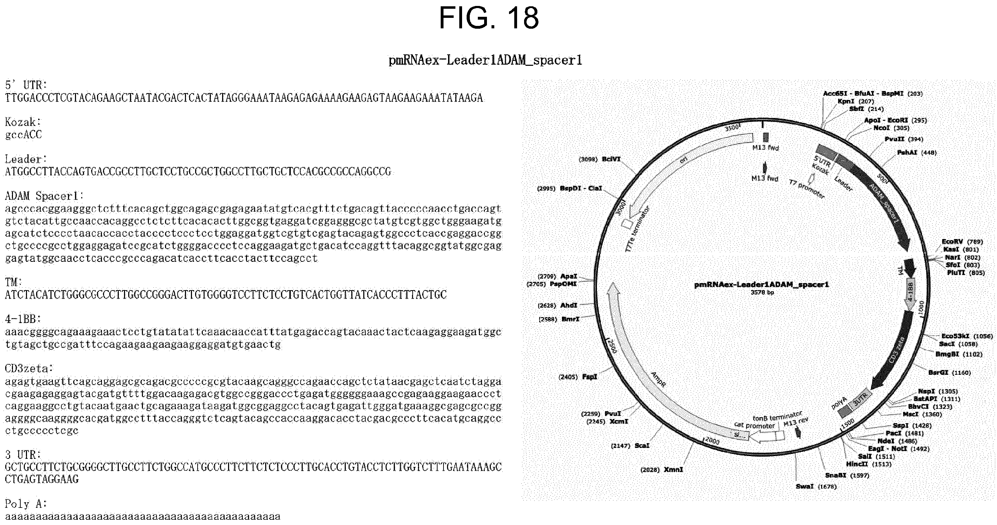

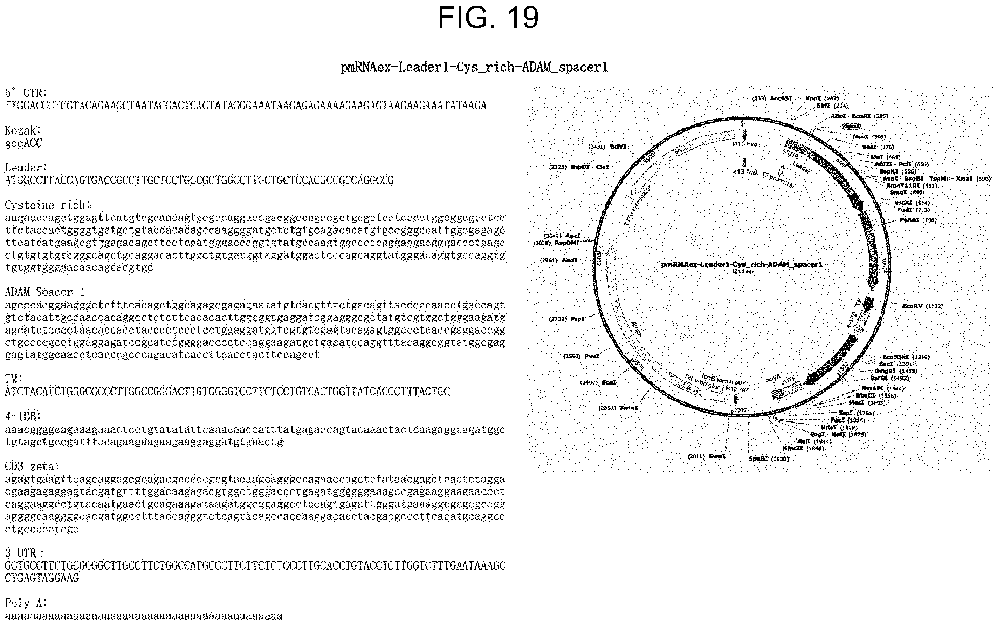

In a BAR which is not part of the claimed invention, the antibody binding domain includes ADAMTS 13. ADAMTS13 is a protein to which a large number of domains are linked, and it is reported that most of antibodies against ADAMTS 13 binds to a spacer domain, and thus it is considered that it is preferable to use the spacer domain as the antibody binding domain. An amino acid sequence of a spacer domain of ADAMTS 13 and a base sequence encoding the same are shown in SEQ ID NOs: 12 and 3 (FIG. 13, FIG. 18). The base sequence encoding the spacer domain of ADAMTS13 may be a substantially homologous base sequence having the identical function. In addition, it is also possible to use a combination of the spacer domain and other portions. One embodiment of the other portions includes a cysteine-rich domain, and an amino acid sequence of the cysteine-rich domain and a base sequence encoding the same are shown in SEQ ID NOs: 13 and 4 (FIG. 14, FIG. 19).

The type of the transmembrane domain b) is not limited as long as it does not inhibit function of the B-cell antibody receptor. For example, transmembrane domains such as CD28, CD3, CD4 and CD8α expressed in T cells or the like can be used. The amino acid sequence or base sequence information of these transmembrane domains is publicly available and can be obtained by keyword search from the NCBI database. In these transmembrane domains, a mutation may be appropriately transferred as long as it does not inhibit the function of the B-cell antibody receptor. Here, the term "mutation" means any mutation including deletion, substitution or addition of one, several or a plurality of amino acids.

An oligopeptide linker or polypeptide linker can optionally be inserted into a binding moiety of the antibody binding domain a), the transmembrane domain b), the intracellular domain of the costimulatory factor c) or the intracellular domain of the activated receptor d), so as to link them to each other. Preferably, a linker composed of 2 to 10 amino acids in length can be used. For example, a flexible linker having an amino acid sequence "GSGGG" can be used.

As one embodiment of the transmembrane domain, the amino acid sequence of the transmembrane domain (TM) of CD3 and the base sequence encoding the same are shown in SEQ ID NO: 15 and SEQ ID NO: 6, respectively.

In the BAR of the present invention, a spacer sequence can be arranged between an extracellular domain and the transmembrane domain or between the intracellular domain and the transmembrane domain. The spacer sequence means any oligopeptide or polypeptide that serves to link the transmembrane domain with the extracellular domain and/or the transmembrane domain with the intracellular domain. The spacer sequence contains up to 300 amino acids, preferably 10 to 100 amino acids, and most preferably 10 to 50 amino acids.

The type of the intracellular domain of the costimulatory factor c) is not particularly limited as long as it is the intracellular domain derived from the costimulatory factor of an immune cell or the like. For example, one or more intracellular domains selected from a group consisting of costimulatory factors such as OX40, 4-1BB, CD27, CD278 and CD28 can be appropriately selected and used. The amino acid sequence or base sequence information of the intracellular domains of these costimulatory factors is publicly available and can be obtained by keyword search from the NCBI database. In the intracellular domains of these costimulatory factors, a mutation may be appropriately transferred as long as it does not inhibit the function of the B-cell antibody receptor.

One embodiment of the intracellular domain of the costimulatory factor includes 4-1BB, and the amino acid sequence thereof and the base sequence encoding the same are shown in SEQ ID NO: 16 and SEQ ID NO: 7, respectively.

The type of the intracellular domain of the activated receptor d) may be appropriately selected depending on the type of immune cells. For example, when a T cell is used as the immune cell, it is preferable to select CD3ζ which is the intracellular domain of a T cell receptor (TCR) (the intracellular domain derived from CD3, also called TCRζ chain). When an NK cell is used, it is preferable to select an intracellular domain derived from NKG2D. The amino acid sequence or base sequence information of these activated receptor domains is publicly available and can be obtained by keyword search from the NCBI database. In these intracellular domains, a mutation may be appropriately transferred as long as it does not inhibit the function of the B-cell antibody receptor.

One embodiment of the intracellular domain of the activated receptor includes CD3ζ, and the amino acid sequence thereof and the base sequence encoding the same are shown in SEQ ID NO: 17 and SEQ ID NO: 8, respectively.

The term "domain" herein is a region in a polypeptide. In the present specification, the "domain" is used as an "antibody binding domain", a "transmembrane domain", and an "intracellular domain", depending on its position in a B-cell antibody receptor molecule.

A signal sequence can also be attached to support transport of BAR protein expressed in the cell to a cell membrane and the like. Examples of the signal sequence include Leader 2 derived from a receptor gene called CSF2RA (SEQ ID NOs: 5 and 14) and Leader 1 derived from CD8 (SEQ ID NOs: 24 and 25).

### [2] Immune cells

As an example of an immune cell into which the B-cell antibody receptor is transfected, an αβT cell, a CD8T cell, a CD4T cell, a γδT cell, an NK cell or the like can be used.

First, peripheral blood mononuclear cells (PBMCs) as raw materials for the αβT cell and the like are prepared. A method for collecting PBMCs is not particularly limited, but for example, PBMCs can be acquired by separating peripheral blood obtained by blood collection using density gradient centrifugation. The amount of blood collected at one time can be appropriately set by a therapeutic method, and for example, when 24 to 72 mL of blood is collected, 1 × 10^7 to 3 × 10^7 PBMCs can be acquired. In addition, when it is necessary to secure a large amount of cells, it is possible to collect mononuclear cell components using a blood component collection device.

In the case of producing αβT cells, collected PBMCs are suspended in a culture solution (medium), and interleukin-2 (IL-2) and an anti-CD3 antibody are added to the obtained cell suspension to perform culture.

The anti-CD3 antibody may be added to the medium or immobilized on the bottom surface of a culture vessel, but it is known that PBMCs can be more suitably cultured by seeding PBMCs in the culture vessel such as a flask in which the anti-CD3 antibody is immobilized (for example, JP-B-3056230). It is preferred that IL-2 is added so as to have a concentration of 1000 to 2000 IU/mL in the medium.

Since the αβT cells prepared as described above contain CD8T cells and CD4T cells, when CD8T cells or CD4T cells are used as immune cells, CD8T cells or CD4T cells can be obtained by positive selection or negative selection from the prepared αβT cells by a cell separation method using magnetic beads or the like.

In the case of producing NK cells, a method of culturing PBMCs in the presence of an agonist of a molecule expressed in NK cells such as an anti-CD16 antibody, IL-2 or the like is common (for example, JP-B-4275680 and the like). Any method may be used to produce NK cells used in the present invention. In addition, CD16+ and/or CD56+ cells prepared by a method described in Patent Literature WO 2009/151183 and the like may be used as the NK cells.

In the case of producing γδT cells, collected PBMCs are cultured in the presence of a phosphate antigen such as bisphosphonate and IL-2 to selectively proliferate and activate γδT cells, so that a cell group containing γδT cells with high purity can be prepared (for example, Patent Literature WO 2006/006720 and the like). Any method may be used to produce γδT cells used in the present invention.

The culture is performed at a temperature of 34 to 38°C, preferably 37°C, and a CO2 concentration of 2 to 10%, preferably 5%, and the culture period is preferably about 1 to 20 days, particularly about 1 to 2 weeks.

The medium to be used is not particularly limited, and commercially available media used for cell culture, such as AIM-V medium (Invitrogen), RPMI-1640 medium (Invitrogen), Dulbecco's modified Eagle's medium (Invitrogen), Iscove's medium (Invitrogen), KBM medium (Khojin Bio), and ALys medium (Cell Science & Technology Institute, Inc.), can be used. The medium may be a medium for human peripheral blood T cells (for example, ALys505N; Cell Science & Technology Institute, Inc.) to which IL-2 is added in advance. In addition, 5 to 20% of Fetal Bovine Serum (FBS), Betal Calf Serum (FCS), Human Serum, Human Plasma (HBP) or the like can be added as necessary.

A gene encoding the B-cell antibody receptor is transfected into the immune cells prepared as described above; αβT cells, CD8T cells, CD4T cells, γδT cells, and/or NK cells. The gene to be transfected can be selected from DNA or RNA. As the transfection method, transfect by a viral vector, transfect by an electroporation method, transfect by lipofection or the like can be appropriately selected.

The immune cells thus prepared are activated by an activation signal and a costimulatory signal by specifically binding to a B cell that produces an antibody causing disease via said antibody by their antibody binding domain a), thereby damaging and eliminating said B cells. As a result, the production of the antibodies causing diseases is suppressed, and the diseases are treated.

### [3] Regenerative medicinal product (cell medicine) and pharmaceutical composition or treatment and/or prevention method using the same

According to the present invention, cells expressing the B-cell antibody receptor (BAR) of the invention can be used for treating and/or preventing a disease, and typically, a regenerative medicinal product (cell medicine) and a pharmaceutical composition can be provided. Here, the "treatment" includes alleviation (moderation) of symptoms or associated symptoms characteristic of the target disease, inhibition or delay of deterioration of symptoms, and the like, and the treatment also includes amelioration of the disease. The "prevention" refers to preventing or delaying the onset/expression of disease (disorder) or symptoms thereof, or reducing risk of onset/expression.

In one embodiment, the regenerative medicinal product (cell medicine) of the present invention contains the cell of the present invention expressing BAR as an active ingredient, and may further contain a suitable excipient or the like.

In another embodiment, the pharmaceutical composition of the present invention contains an effective amount of the cells of the present invention as an active ingredient, and may further contain a suitable pharmaceutically acceptable excipient or the like. Examples of the excipient contained in the cell preparation and the pharmaceutical composition include various cell culture media, phosphate buffered saline, isotonic saline, and the like.

The term "regenerative medicinal product" in the present specification refers to:
1) cells of human or animal subjected to culture or other processing, among substances intended to be used for
   (i) reconstruction, restoration or formation of a structure or function of a human or animal body; or
   (ii) treatment or prevention of a human or animal disease; or
2) substances transferred into human or animal cells and containing genes expressed in these bodies, among substances intended to be used for treatment of a human or animal disease. Although not particularly limited, examples of the regenerative medicine product include:
   1) human cell processing products
      a human somatic cell processing product
      b human somatic stem cell processing product
      c human embryonic stem cell processing product
      d human induced pluripotent stem cell processing product
   2) animal cell processing products
      a animal somatic cell processing product
      b animal somatic stem cell processing product
      c animal embryonic stem cell processing product
      d animal induced pluripotent stem cell processing product
   3) gene therapy products
      a plasmid vector product
      b viral vector product
      c gene expression therapeutic product (excluding a and b).

A regenerative medicinal product containing the immune cell expressing the B-cell antibody receptor of the present invention as an active ingredient corresponds to a human somatic cell processing product.

### [4] Nucleic acid encoding the B-cell antibody receptor (BAR) of the present invention

According to the present invention, a nucleic acid encoding an amino acid sequence of the BAR of the present invention is provided. Unless otherwise specified, the "nucleic acid (or base sequence) encoding the amino acid sequence" includes all base sequences that are polycondensed each other and encode the same amino acid sequence.

The nucleic acid encoding the BAR can be easily prepared from a cDNA sequence of a specified responsible enzyme by a conventional method. For example, the nucleic acid encoding a), among a) to d) constituting BAR, can be amplified and acquired, for example, by extracting mRNA from somatic cells, preparing cDNA from the mRNA by reverse transcription, and using a PCR method or the like using the cDNA as a template. The nucleic acids encoding b), c) and d) can be amplified and acquired, for example, by extracting mRNA from immune cells, preparing cDNA from the mRNA by reverse transcription, and using a PCR method or the like using the cDNA as a template.

The nucleic acid encoding the BAR can be obtained by linking the nucleic acids encoding each of domains acquired as described above. The nucleic acids encoding each of domains can be linked using a known method such as cloning by homologous recombination, an overlap extension method, or the like.

The nucleic acid of BAR prepared can be incorporated into a plasmid or a viral vector and used, or mRNA can be synthesized and used.

### [5] Method for producing immune cells expressing the B-cell antibody receptor (BAR) of the present invention

A method for producing immune cells expressing the BAR of the present invention includes a step of transfecting a nucleic acid encoding the BAR according to the above [4] into immune cells. The step is performed by a method of transforming immune cells in vitro using a viral vector or non-viral vector containing a nucleic acid encoding the BAR, or an electroporation method.

For example, they can be produced by transforming immune cells in vitro using a viral vector or plasmid vector containing the nucleic acid encoding the BAR according to the present invention or the nucleic acid itself encoding the BAR according to the present invention.

The type and use of the vector are not particularly limited, but the vector may be a plasmid vector or a viral vector (for example, lentivirus, adenovirus, or retrovirus). Also, the vector may be, for example, a cloning vector or an expression vector. Examples of the expression vector include vectors for prokaryotic cells such as E. coli or actinomycetes, or vectors for eukaryotic cells such as yeast cells, insect cells, or mammalian cells.

Device and method used for electroporation are not particularly limited, and MaxCyte GT (MaxCyte, Inc.), Nepa Gene (Nepa Gene Co., Ltd.), Nucleofector (Lonza KK.), Gene Pulser (Bio-Rad Laboratories, Inc.) or the like can be used.

The immune cell expressing the B-cell antibody receptor (BAR) recognizes an antibody on a B cell with the antibody binding domain a), then transmits its recognition signal to the inside of a T cell or the like through a ζ chain, and activates a signal for inducing cytotoxic activity in the cell through the transmembrane domain and the costimulatory factor, thereby exerting cytotoxic activity on the B cell that produces the antibody causing disease.

### [6] Immune cells expressing B-cell antibody receptor (BAR) of the present invention

The immune cells expressing the BAR of the present invention is immune cells into which the nucleic acid encoding BAR of the invention is transfected and expressed by the production method of the above [5].

The regenerative medicinal product (cell medicine) of the present invention is an injection (cell suspension) obtained by suspending the immune cells of the present invention in a liquid that can be used as a medicine (for example, physiological saline), and can be used for treating and/or preventing a disease. This injection may be injected intravenously, intradermally, subcutaneously or the like, may be directly injected into a lesion, or may be systemically administered as a drip. The injection may contain a physiologically acceptable carrier or excipient.

The number of immune cells contained in the regenerative medicinal product (cell medicine) of the present invention can be appropriately set according to administration method, type of disease, symptom of the patient, age, sex, and weight of the patient, severity of the disease, and the like. Normally, the number may be set at 10^8 to 10^12 cells/person (preferably 10^9 cells/person).

A method for producing the regenerative medicinal product (cell medicine) of the present invention is not particularly limited. For example, 1) the immune cells of the present invention are collected from the medium by centrifugation or the like; 2) the collected immune cells are washed with a washing liquid (for example, physiological saline, PBS, or the like); 3) the washed immune cells are collected from the washing liquid by centrifugation or the like; and 4) the collected immune cells are suspended in a liquid that can be used as a medicine (for example, physiological saline), whereby the regenerative medicinal product (cell medicine) can be produced as a drip infusion capable of being intravenously administered.

The disease to be a target of the present invention is Fabry disease. Other examples of a disease to be a target of the regenerative medicinal product (cell medicine) include lysosomal diseases such as Gaucher disease, Pompe disease, and mucopolysaccharidosis. In addition, examples of the disease include diseases to which replacement therapy is adopted, such as hemophilia and thrombotic thrombocytopenic purpura.

A subject to be administered with the regenerative medicinal product (cell medicine) of the present invention is, for example, an animal suffering from or likely to suffer from the above disease. The animal is, for example, a mammal, preferably a human.

Hereinafter, the present invention will be described in details with reference to Examples, but the present invention is not limited to these Examples.

### EXAMPLES

### 1. Identification of base sequence and amino acid sequence of GLA

The base sequence and amino acid sequence of GLA are publicly available, and the inventors obtained sequence information from the National Center for Biotechnology Information NCBI database (https://www.ncbi.nlm.nih.gov/protein/4NXS_A). The base sequence of D1 domain which is an antibody recognition sequence region was identified according to Non Patent Literature (J Mol Biol.2004 Mar 19;337(2):319-35.). The base sequence encoding the GLA full-length protein is shown in SEQ ID NO: 1, and the base sequence encoding the antibody recognition sequence region (D1 domain) of GLA is shown in SEQ ID NO: 2. MGC Human GLA Sequence-Verified cDNA (Clone ID: 3609235) (Dharmacon Inc.) in which the sequence of GLA was inserted into a plasmid was purchased, and the plasmid was used as a template to isolate the base sequence of the full-length protein (Full Length) using primer sets shown in SEQ ID NO: 19 and SEQ ID NO: 20 and the base sequence of the antibody recognition sequence region (D1 region) using primer sets shown in SEQ ID NO: 19 and SEQ ID NO: 21.

### 2. Specification of base sequences of two types of BARs

As amino acid sequences of the intracellular domain of the costimulatory factor (4-1BB) and the intracellular domain of the activated receptor (CD3ζ), the transmembrane domain (TM) and the spacer sequence which constitute BAR, those that have already been demonstrated to be effective in CD19 CAR-T cells (Patent Literature; WO 2012079000 A1 SEQ ID NO: 8) were employed. The base sequence in which the transmembrane domain, 4-1BB and CD3ζ were bound was synthesized by an artificial gene synthesis contract service of Integrated DNA Technologies, Inc., and the base sequence encoding the transmembrane domain, 4-1BB and CD3ζ was acquired using the synthesized base sequence as a template and using primer sets shown in SEQ ID NO: 22 and SEQ ID NO: 23.

The base sequence of the full-length protein (Full Length) or the antibody recognition sequence region (D1 region) described above and the base sequence in which the transmembrane domain, 4-1BB and CD3ζ were bound were inserted into Xhol-EcoRI of the pMSCV plasmid by In-fusion cloning, and the base sequence of the BAR was cloned.

### 3. Preparation of GLA BAR-T cell

The base sequences of the two types of BARs specified in the above item 2 are subcloned into a general-purpose plasmid, and a plurality of restriction enzyme sites are transfected into the 5' side of Spacer of this sequence. The sequence of an antigen was recombined using this restriction enzyme site. The base sequence of the full-length protein of GLA or D1 region, which is the antibody binding domain, was amplified by PCR, and after confirming that no mutation was contained in the sequence by Sequencing, recombined into the plasmid.

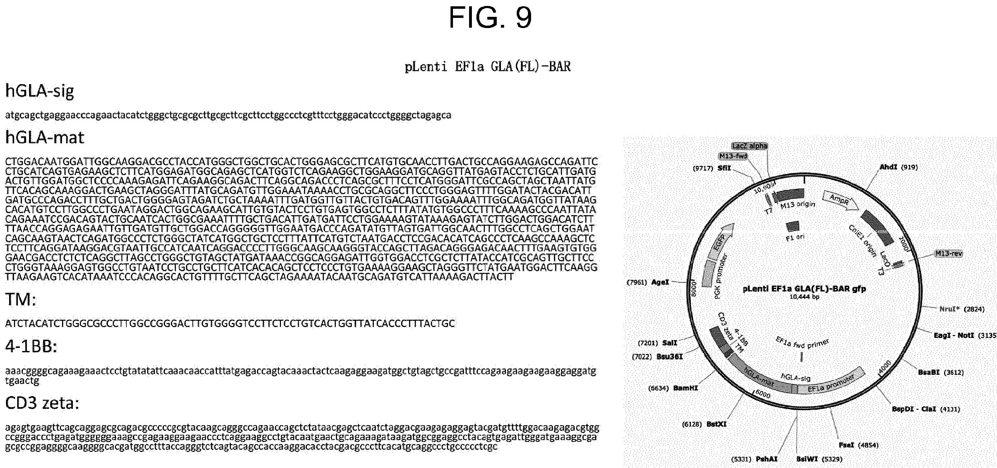

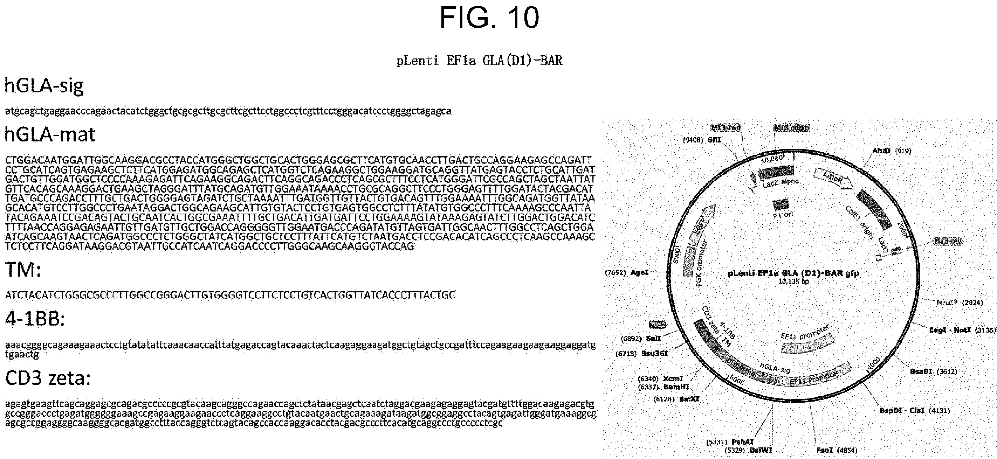

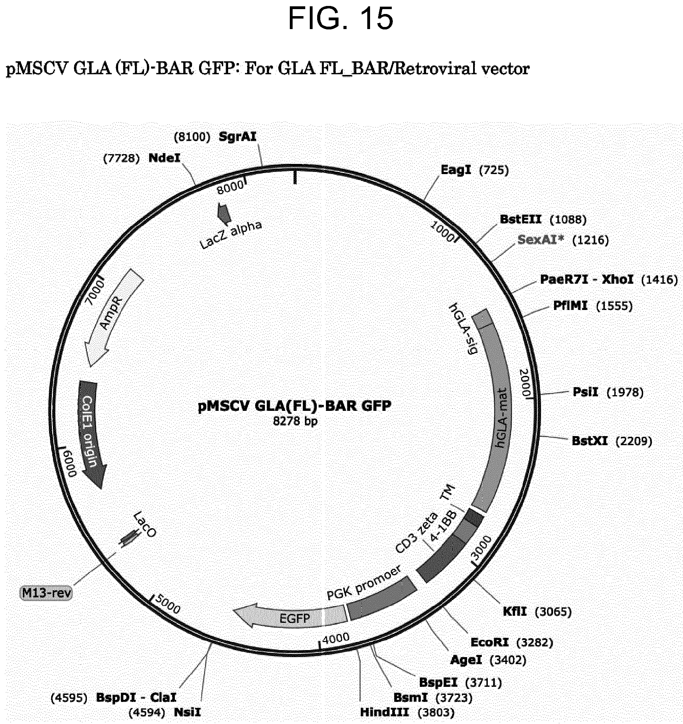

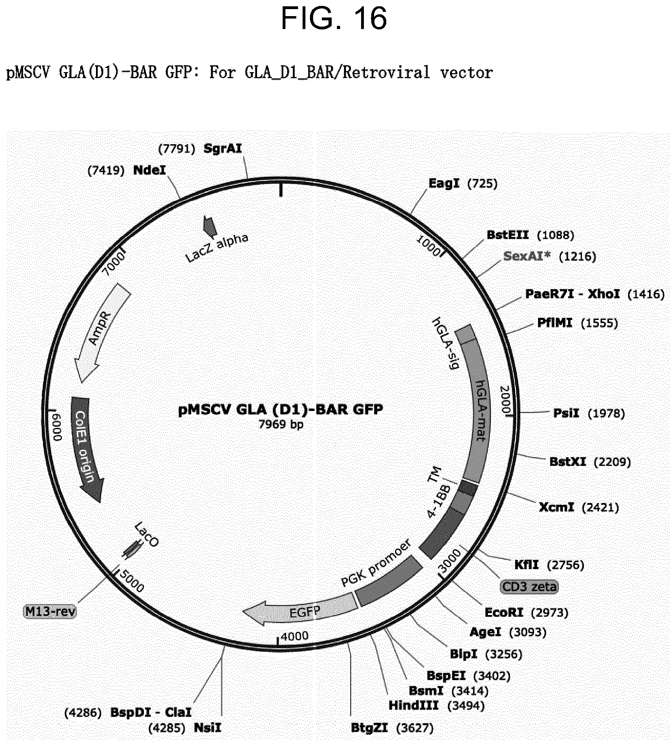

Using thus obtained plasmid subjected to recombination as described above, the base sequence encoding the full-length protein (Full Length: FL) BAR of GLA and the base sequence encoding GLA BAR of the D1 region were inserted into retroviral vectors (FIGS. 15 and 16) or lentiviral vectors (FIG. 9, FIG. 10), respectively, to prepare viral vectors.

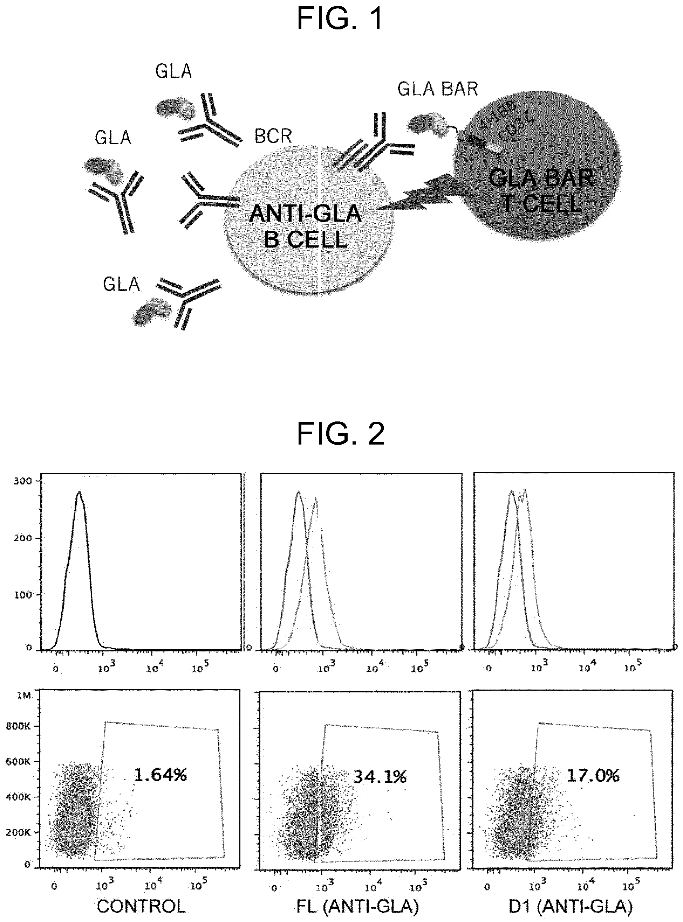

Using these vectors, transfection into human T cells was performed to prepare FL BAR-T cells and D1 BAR-T cells. FACS was performed using an anti-GLA antibody. The results are shown in FIG. 2. A rightward shift of mean fluorescence intensity was observed, and it can be determined that the BAR was expressed and the fluorescence intensity was enhanced in the FL BAR-T cells and the D1 BAR-T cells.

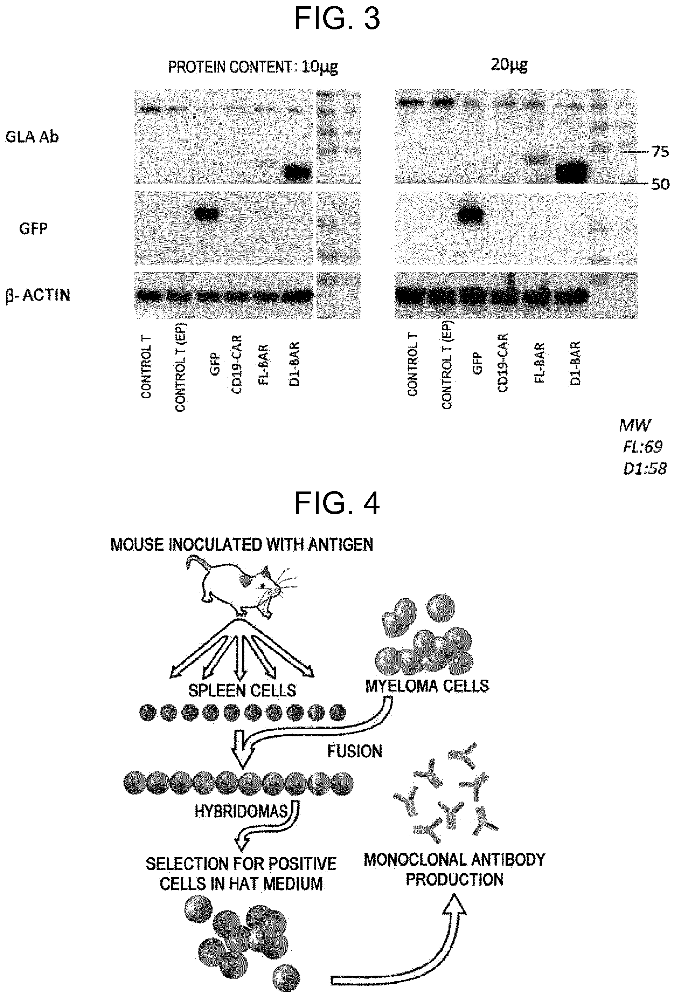

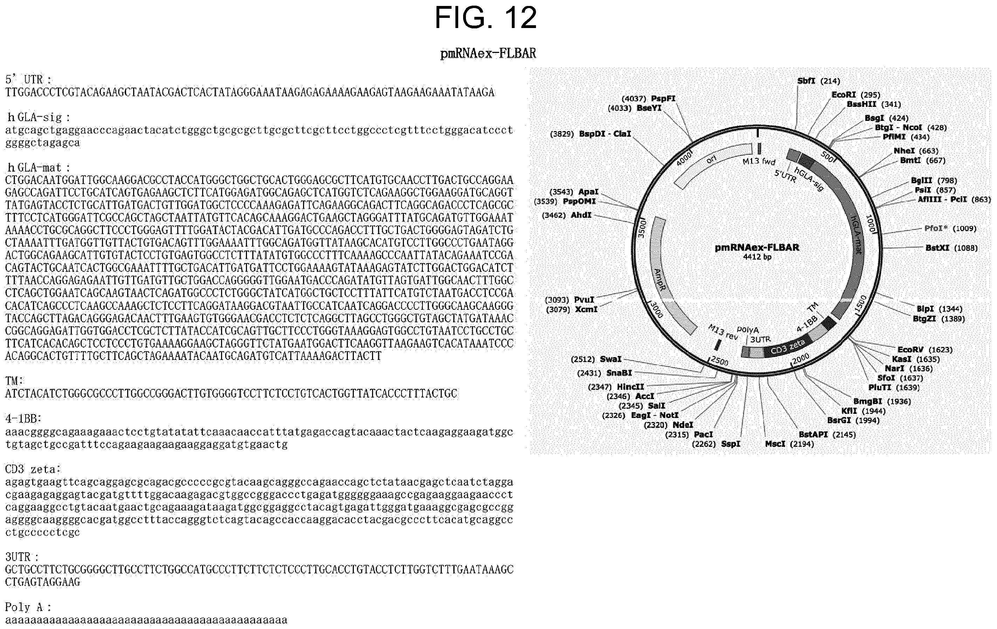

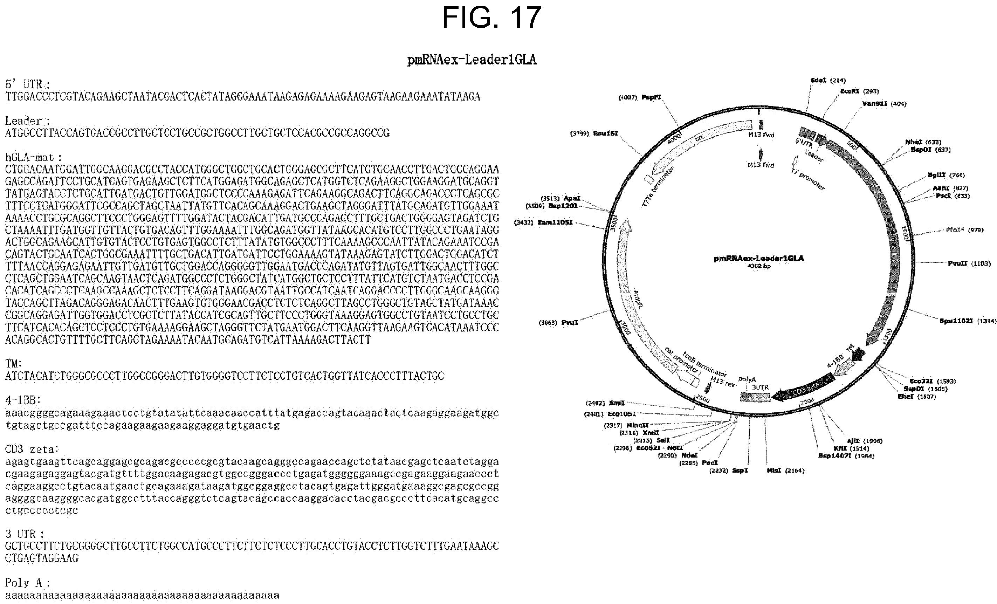

As another transfection method, mRNA Transfection was performed. The base sequence constituting the BAR was subcloned into a plasmid vector (pmRNAex, FIG. 12, FIG. 17) for performing In vitro transcription, and a BAR mRNA fragment was isolated using mMESSAGE mMACHINE T7 Ultra kit, Catalog# AM1345 (Thermo Fisher Science). An mRNA encoding the BAR was transfected into human T cells by an electroporation device of MaxCyte, Inc., and protein expression level was confirmed by Western blotting. After 14 hours from electroporation, the expression level of the BAR was measured using β-actin as a housekeeping protein. From this result, it was confirmed that the BAR was expressed by transfecting mRNA of GLA BAR into human T cells by an electroporation method. The BAR using the D1 region as the antibody binding domain showed extremely strong expression as compared with the BAR using FL as the antibody binding domain (FIG. 3).

### 4. Preparation of anti-GLA antibody-overexpressed cells (anti-GLA scFv-overexpressing M1 cells)

Spleen cells of a mouse inoculated with human GLA protein and myeloma cells were fused to obtain hybridomas. This heterogeneous hybridomas population was positively selected in a HAT medium to obtain hybridomas that produce an anti-GLA monoclonal antibody (FIG. 4).

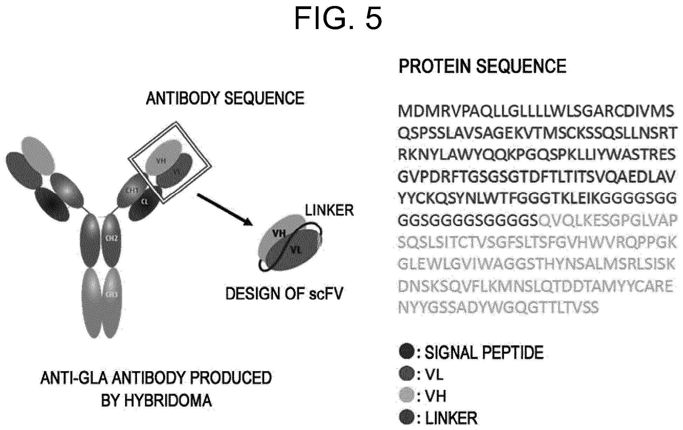

Next, antibody sequencing was performed from the anti-GLA monoclonal antibody-producing B cells to obtain amino acid sequences of variable regions of heavy chain and light chain of the anti-GLA monoclonal antibody. Based on the obtained amino acid sequence, a linker was interposed to create a sequence of scFv of the anti-GLA antibody (FIG. 5). The amino acid sequence of the scFv of the anti-GLA antibody and the base sequence encoding the same are shown in SEQ ID NO: 18 and SEQ ID NO: 9, respectively.

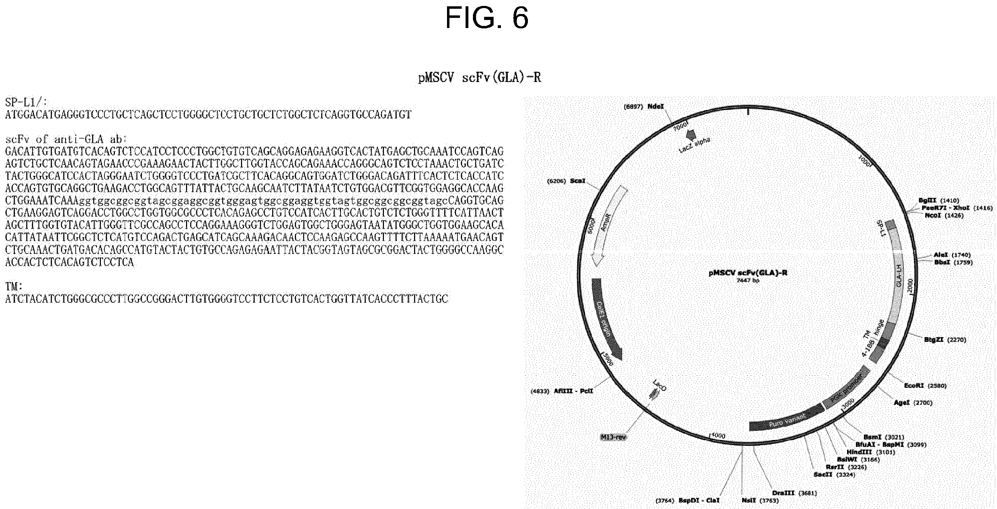

The base sequence of the scFv of the anti-GLA antibody specified above was subcloned, and recombined into the plasmid vector (FIG. 6).

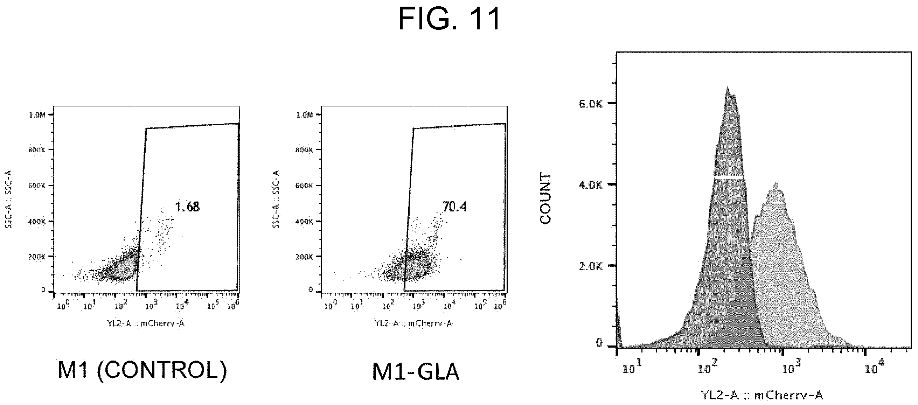

The obtained plasmid vector was subcloned, and recombined into a retroviral vector. M1 cells that are established cells derived from mouse myelogenous leukemia were infected using the obtained retroviral vector, thereby creating Transfectant in which the scFv of the anti-GLA antibody was forcibly expressed, and the Transfectant was used as a model cell of neutralizing antibody-producing B cells. It was confirmed from a binding assay of GLA-mCherry for the prepared model cells that the anti-GLA antibodies were overexpressed (FIG. 11).

### 5. Preparation of CD19 CAR-T cells

According to Material and Method of Non Patent Literature (New England Journal of Medicine 2014; 371 (16): 1507-1517), a base sequence encoding CD19 CAR-T described in Patent Literature (WO 2012079000 A1 SEQ ID NO: 8) was inserted into a retroviral vector or a lentiviral vector. After preparing the retroviral vector or the lentiviral vector, transfection into human T cells was performed using retronectin to prepare CD19 CAR-T cells.

### 6. In Vitro killing assay

### (Method)

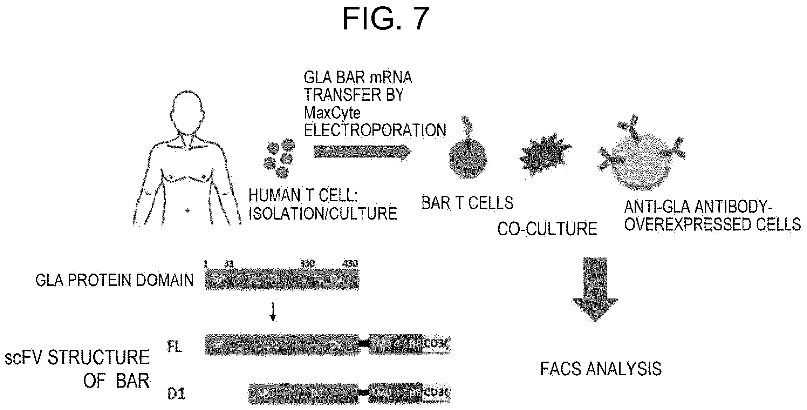

FIG. 7 shows an outline of a killing assay on the two types of GLA BAR-T cells prepared in the above item 2 and the M1 cells forcibly expressing the scFv of the anti-GLA antibody prepared in the above item 3. The "FL BAR-T cells" in which the full-length protein of GLA was adopted as the antibody binding domain and the "D1 BAR-T cells" in which the D1 region of GLA was adopted as the antibody binding domain were used as examination targets. The immune cells are human primary T cells, and the T cell culturing method and the protocol of the killing assay are the same as those in the experiment in validation of CD19 CAR-T cells (Non Patent Literature: Frontiers in immunology 8, 1956 (2018) or Non Patent Literature: Science 353, 179-184 (2016)). In the method, a mononuclear cell fraction was acquired from human peripheral blood by specific gravity centrifugation, and transferred to a plate to which an anti-CD3/CD28 antibody was adhered to promote selective proliferation of T cells. On the next day, differentiation of T cells into effector cells was suppressed and proliferation stimulation was given while maintaining Memory (Stem) cells with IL-7: 20 ug/ml and IL-15: 10 ug/ml. This cytokines combination has been reported as the combination in which cytotoxic activity of CAR-T cells was most strongly maintained and the cytotoxic activity was most well maintained even when administered in vivo. On day 2, gene transfection into stimulated human T cells was performed by a recombinant retrovirus encoding an anti-CD19 CAR or a BAR, and on day 5, co-culture with M1 cells expressing the scFv of the anti-GLA antibody and M1 cells into which GFP as an infection control was transfected was started, and analysis was performed by FACS on day 0 and day 4.

The co-culture of the BAR-T cells and the M1 cells transfected with GFP was subjected as a transfection control, and the co-culture of the CD19 CAR-T cells and the scFv-overexpressing M1 cells of the anti-GLA antibody was subjected to eliminate a possibility of a non-specific reaction.

### (Results)

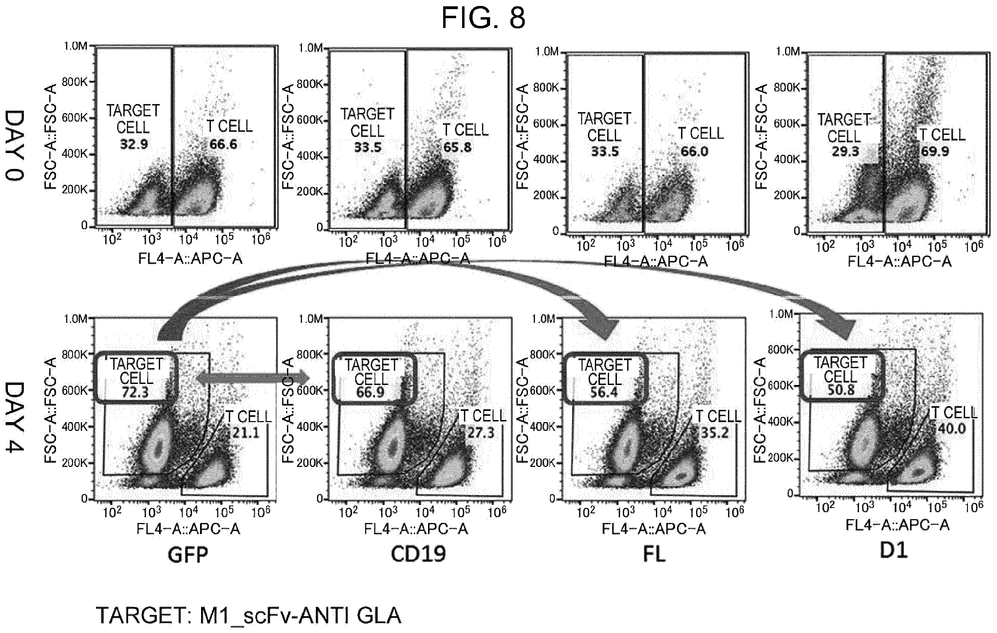

The results are shown in FIG. 8. Analysis by FACS was performed 4 days after the start of co-culture. In X-axis, the co-cultured cells were stained with APC anti-CD3 antibody and subjected to FACS. A cell viability rate of M1 cells showed 72.3% in the co-culture of the GFP-transfected M1 cells and the BAR-T cells as a control (GFP in the lower part of FIG. 8), and showed 66.9% in the co-culture of the M1 cells overexpressing the scFv of the anti-GLA antibody and the CD19 CAR-T cells (CD19 in the lower part of FIG. 8), and it was confirmed that there was no non-specific reaction. On the other hand, a viable cells rate of M1 cells showed 56.4% in the co-culture of the M1 cells overexpressing the scFv of the anti-GLA antibody and the FL BAR-T cells (FL in the lower part of FIG. 8), and showed 50.8% in the co-culture of the M1 cells overexpressing the scFv of the anti-GLA antibody and the D1 BAR-T cells (D1 in the lower part of FIG. 8). The number of M1 cells was significantly reduced as compared with the control. In addition, from the results of this killing assay and the strength and stability of protein expression, it was determined that the GLA BAR-T cells having the D1 region as an extracellular domain are more effective for specific removal of anti-GLA antibody-producing B cells.

### 7. Preparation of target cells (anti-GLA scFv-expressing cells)

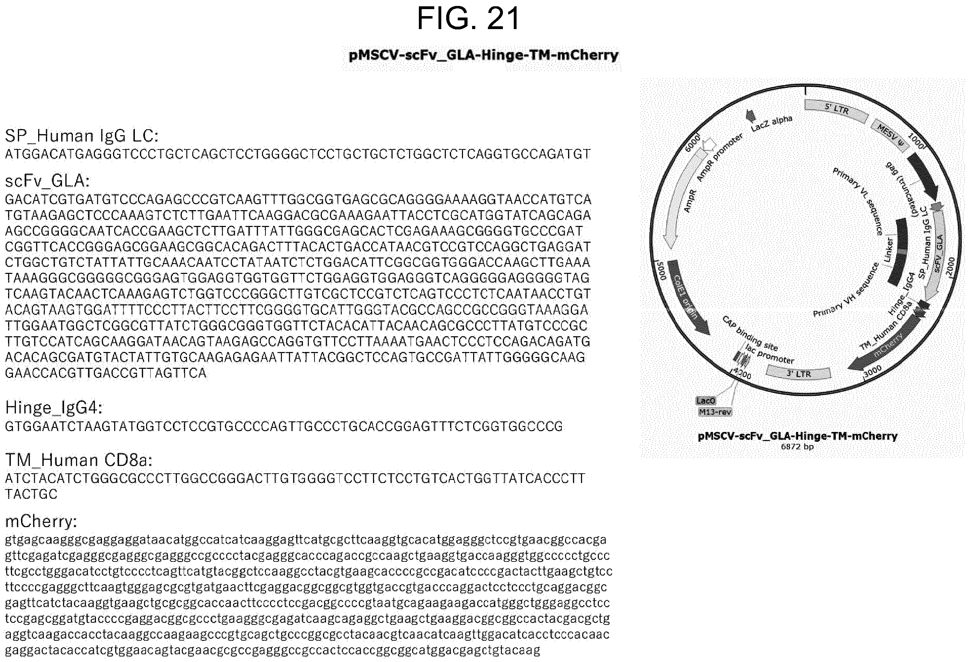

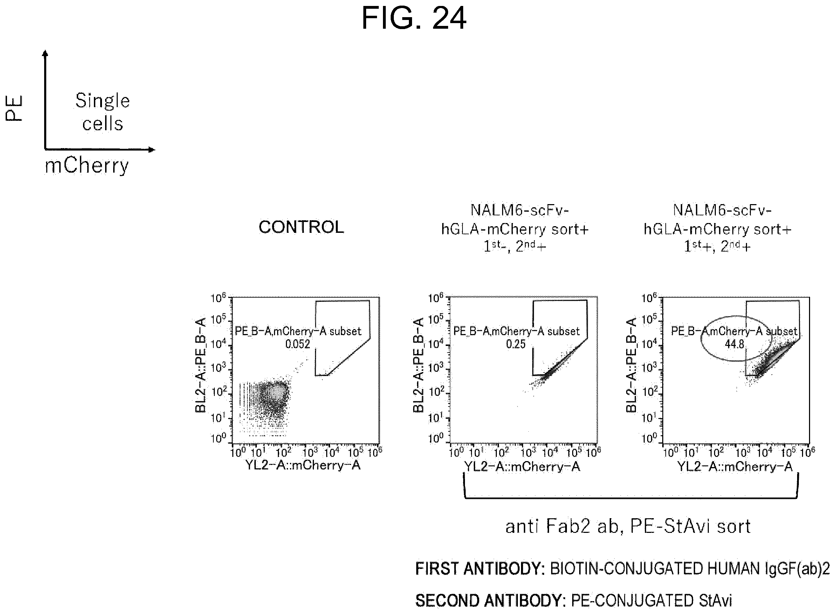

A retroviral vector encoding scFv of the anti-GLA antibody (FIG. 21) was transfected into Nalm6 (RCB1933) (purchased from RIKEN Cell Bank), which is a human precursor B cell line of leukemia, to prepare target model cells of BAR-T cells. After transfection of the retroviral vector, the cells were sorted by mCherry, reacted with a biotin-conjugated anti-Fab2 antibody, and then reacted with fluorescently labeled streptavidin, and analyzed. As a result, expression of the anti-GLA antibody by the generated target model cells was confirmed (FIG. 24).

### 8. Preparation of GLA BAR-T cells

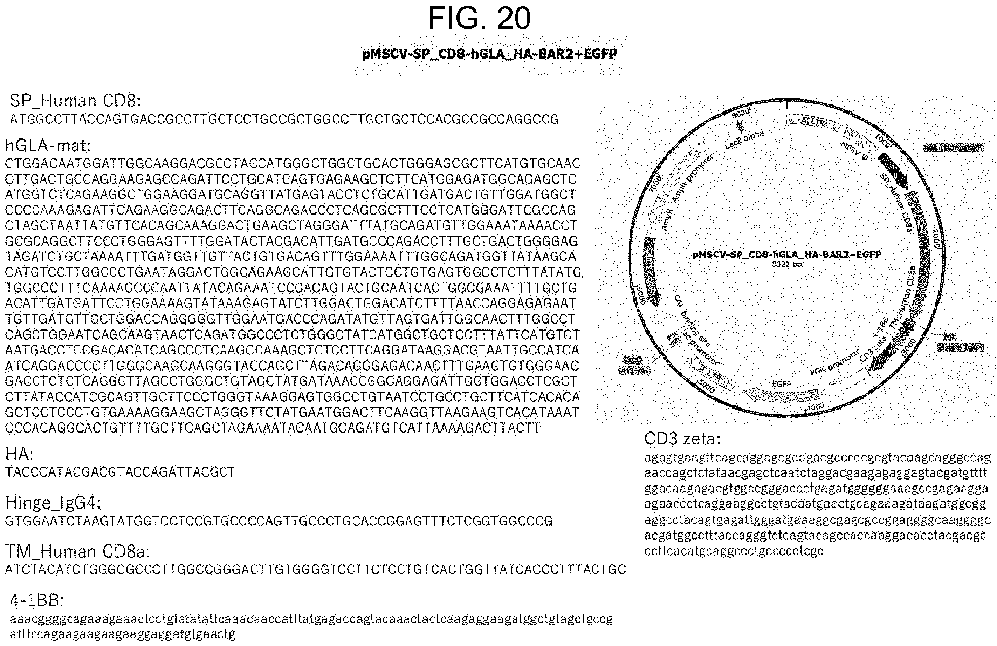

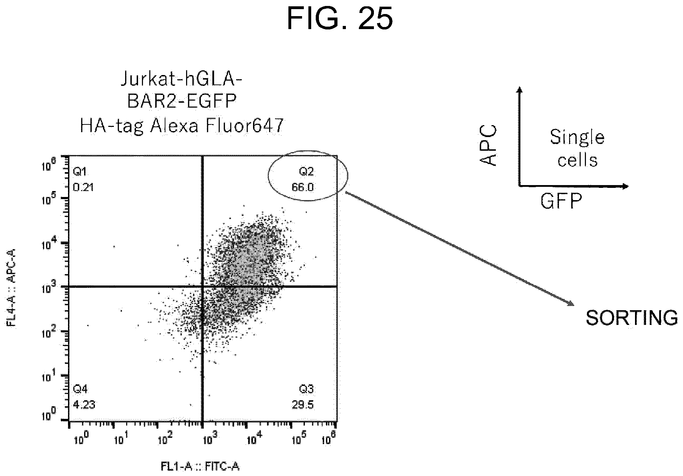

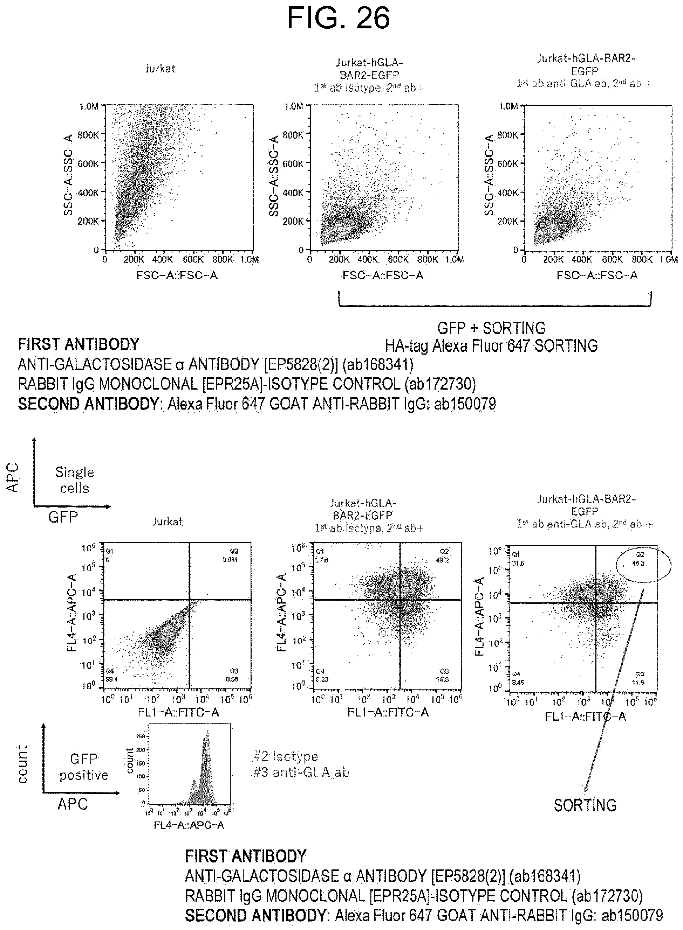

A retroviral vector (FIG. 20) encoding an amino acid sequence of hGLA (SEQ ID NO: 10; DNA sequence is shown in SEQ ID NO: 1) was transfected into Jurkat, which is a human leukemia T cell line, to prepare BAR-T cells. After transfection with the retroviral vector, the cells were sorted by GFP (FIG. 25), reacted with an anti-GLA antibody, and then reacted with a fluorescent-labeled secondary antibody. As a result of analysis, expression of BAR was confirmed (FIG. 26).

### 9. Confirmation of activation of BAR-T cells by mixed culture

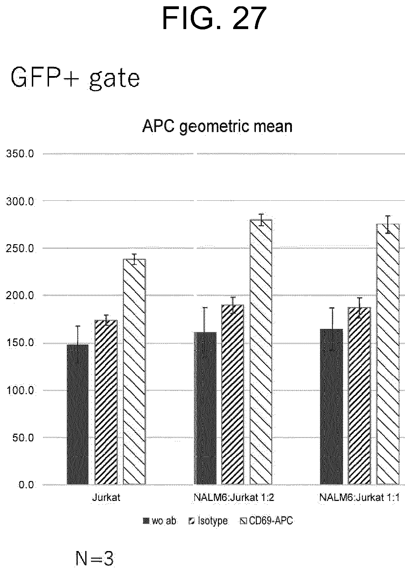

The BAR-T cells (Jurkat-GLA-BAR2-EGFP: Effector) prepared in the above item 8 and the target cells (NALM6-scFv-GLA-mCherry: Target) prepared in the above item 7 were mixed and cultured at mixing ratios of 1 : 0, 2 : 1, 1 : 1 for 24 hours, and a detection test of CD69 was performed. It is known that expression of CD69 increases when Jurkat is activated, and it was considered that the expression of CD69 increases depending on the mixing ratio, if the BAR-T cell transfected into Jurkat binds to the scFv of the anti-GLA antibody of the target cells and an activation signal is received. As a result of performing the mixed culture, the expression of CD69 increased in the presence of the target cells as compared with a control in the absence of the target cells (E : T = 1 : 0), and the expression of CD69 further increased when the target cells increased (E : T = 2 : 1 → 1 : 1) (FIG. 27). From this, it was confirmed that the BAR-T cells were activated in an antigen-specific manner.

### 10. Preparation of BAR-T cells (ADAMTS13) (not covered by the claims)

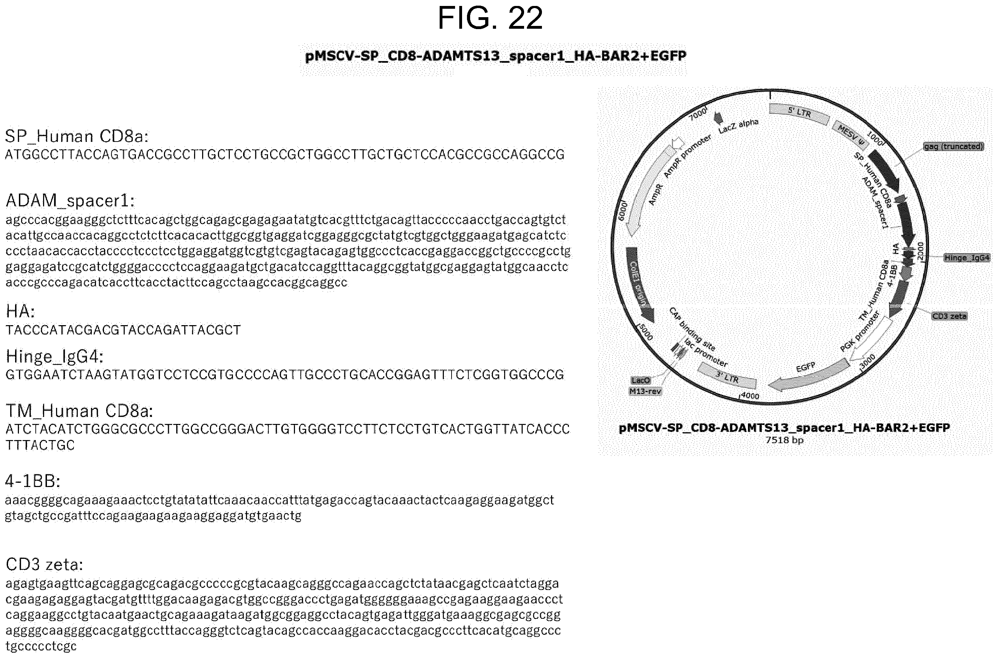

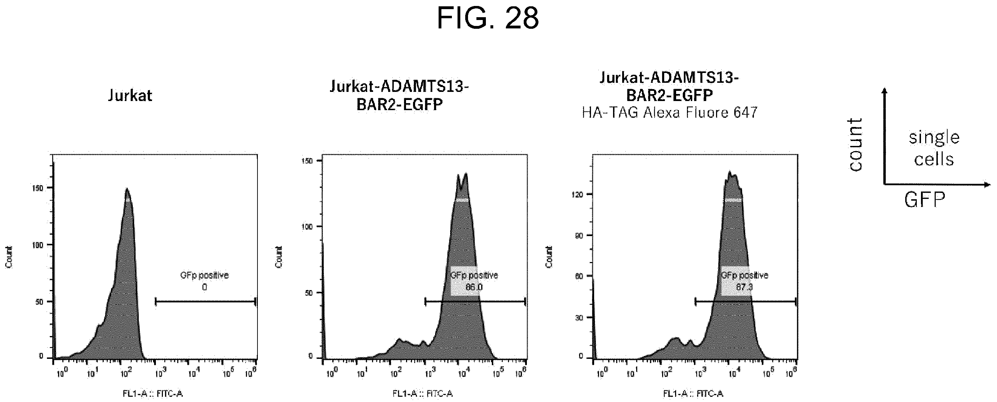

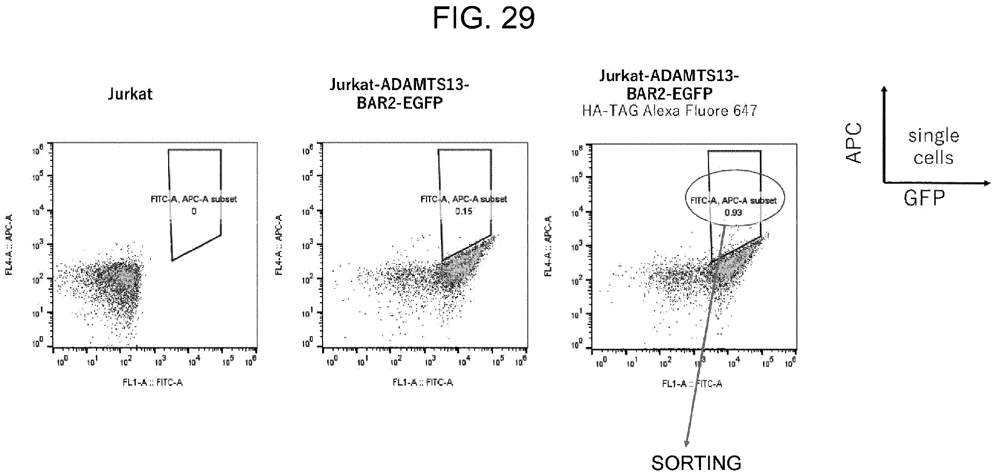

A retroviral vector encoding the amino acid sequence of ADAMTS13 (FIG. 22) was transfected into Jurkat, which is a human leukemia T cell line, to prepare BAR-T cells. After transfection with the retroviral vector, the cells were sorted by GFP, and expression of ADAMTS13 was confirmed by HA-TAG (FIGS. 28 and 29).

### INDUSTRIAL APPLICABILITY

By using the present invention, it is possible to specifically damage and eliminate B cells that produce a specific antibody, and thus it can be expected to improve patients with lysosomal storage disease or the like who produce an antibody against the enzyme and cannot obtain a sufficient therapeutic effect even though enzyme replacement therapy is performed. The present invention can also be expected as a therapeutic agent for diseases caused by antibodies.

### SEQUENCE LISTING FREE TEXT

SEQ ID NO: 1 <223> DNA encoding α-GAL full-length protein
SEQ ID NO: 2 <223> DNA encoding α-GAL D1 region protein
SEQ ID NO: 3 <223> DNA encoding ADAMTS13 spacer domain
SEQ ID NO: 4 <223> DNA encoding ADAMTS13 cysteine-rich domain
SEQ ID NO: 5 <223> DNA encoding signal sequence Leader 2
SEQ ID NO: 6 <223> DNA encoding transmembrane domain
SEQ ID NO: 7 <223> DNA encoding 4-1BB
SEQ ID NO: 8 <223> DNA encoding CD3ζ
SEQ ID NO: 9 <223> DNA encoding scFv of anti-GLA antibody
SEQ ID NO: 10 <223> α-GAL full-length protein
SEQ ID NO: 11 <223> α-GAL D1 region protein
SEQ ID NO: 12 <223> ADAMTS13 Spacer domain
SEQ ID NO: 13 <223> ADAMTS13 Cysteine-rich domain
SEQ ID NO: 14 <223> Signal sequence Leader 2 protein
SEQ ID NO: 15 <223> Transmembrane domain protein
SEQ ID NO: 16 <223> 4-1BB protein
SEQ ID NO: 17 <223> CD3ζ protein
SEQ ID NO: 18 <223> scFv protein of anti-GLA antibody
SEQ ID NO: 19 <223> α-GAL Primer (forward)
SEQ ID NO: 20 <223> α-GAL Primer (reverse: full length)
SEQ ID NO: 21 <223> α-GAL Primer (reverse: D1 region)
SEQ ID NO: 22 <223> TM, 4-1BB, CD3ζ Primer (forward)
SEQ ID NO: 23 <223> TM, 4-1BB, CD3ζ Primer (reverse)
SEQ ID NO: 24 <223> DNA encoding signal sequence Leader 1
SEQ ID NO: 25 <223> Signal sequence Leader 1 protein
SEQ ID NO: 26 <223> DNA encoding human CD8 signal peptide
SEQ ID NO: 27 <223> Human CD8 signal peptide
SEQ ID NO: 28 <223> DNA encoding HA
SEQ ID NO: 29 <223> HA protein
SEQ ID NO: 30 <223> DNA encoding IgG4 antibody hinge region
SEQ ID NO: 31 <223> IgG4 antibody hinge region protein
SEQ ID NO: 32 <223> DNA encoding human IgG anti-GLA antibody L chain
SEQ ID NO: 33 <223> Human IgG anti-GLA antibody L-chain protein
SEQ ID NO: 34 <223> DNA encoding mCherry
SEQ ID NO: 35 <223> mCherry protein
SEQ ID NO: 36 <223> DNA encoding scFv of anti-GLA antibody
SEQ ID NO: 37 <223> DNA encoding ADAMTS13 spacer domain
SEQ ID NO: 38 <223> ADAMTS13 Spacer domain
SEQ ID NO: 39 <223> DNA encoding signal peptide of human IgG anti-ADAMTS13 antibody L-chain
SEQ ID NO: 40 <223> Signal peptide of human IgG anti-ADAMTS 13 antibody L-chain
SEQ ID NO: 41 <223> DNA encoding scFv of anti-ADAMTS 13 antibody
SEQ ID NO: 42 <223> scFv of anti-ADAMTS 13 antibody
SEQ ID NO: 43 <223> DNA encoding human CD8a transmembrane domain
SEQ ID NO: 44 <223> Human CD8a transmembrane domain

## Claims

1. A B-cell antibody receptor wherein an antibody binding domain a), a transmembrane domain b), an intracellular domain of a costimulatory factor c), and an intracellular domain of an activated receptor d) are combined, wherein the antibody binding domain a) comprises:
- a full-length protein of α-GAL, and the base sequence encoding the full-length protein of α-GAL comprises a base sequence set forth in SEQ ID NO: 1, or a base sequence 95% or more identical to the base sequence set forth in SEQ ID NO: 1, having an identical function; or
- an antibody recognition sequence region (D1 region) of α-GAL, and the base sequence encoding the antibody recognition sequence region (D1 region) of α-GAL comprises a base sequence set forth in SEQ ID NO: 2, or a base sequence 95% or more identical to the base sequence set forth in SEQ ID NO: 2, having an identical function.

2. The B-cell antibody receptor according to claim 1, wherein a linker is inserted into a binding moiety of said a), b), c) or d).

3. The B-cell antibody receptor according to claim 1 or 2, wherein the transmembrane domain b) is selected from a group consisting of CD28, CD3ζ, CD4, and CD8α.

4. The B-cell antibody receptor according to any one of claims 1 to 3, wherein the intracellular domain of a costimulatory factor c) is selected from a group consisting of OX40, 4-1BB, CD27, CD278, and CD28.

5. The B-cell antibody receptor according to any one of claims 1 to 4, wherein the activated receptor d) is selected from a group consisting of TCR and NKG2D.

6. A nucleic acid encoding the B-cell antibody receptor according to any one of claims 1 to 5.

7. A vector comprising the nucleic acid according to claim 6.

8. An immune cell expressing a B-cell antibody receptor that has been transfected using the nucleic acid according to claim 6 or the vector according to claim 7.

9. The immune cell according to claim 8, wherein the immune cell is an αβT cell, a CD8T cell, a CD4T cell, a γδT cell, or an NK cell.

10. A regenerative medicinal product comprising the immune cell according to claim 8 or 9 as an active ingredient.

11. An immune cell expressing a B-cell antibody receptor according to any one of claims 1 to 5 for use in a method of treating and/or preventing lysosomal storage disease.

12. The immune cell for use according to claim 11, wherein the lysosomal storage disease is Fabry disease.

## Patentansprüche

1. B-Zell-Antikörper-Rezeptor, wobei eine Antikörper-Bindungsdomäne a), eine Transmembrandomäne b), eine intrazelluläre Domäne eines kostimulatorischen Faktors c) und eine intrazelluläre Domäne eines aktivierten Rezeptors d) kombiniert werden, wobei die Antikörper-Bindungsdomäne a) Folgendes umfasst:
- ein vollständiges Protein α-GAL, und die Basensequenz, die das vollständige Protein α-GAL codiert, umfasst eine Basensequenz, die in SEQ ID NO: 1 dargelegt ist, oder eine Basensequenz, die 95% oder mehr mit der Basensequenz identisch ist, die in SEQ ID NO: 1 dargelegt ist, und eine identische Funktion aufweist; oder
- eine Antikörper-Erkennungssequenz-Region (D1-Region) von α-GAL, und die Basensequenz, die die Antikörper-Erkennungssequenz-Region (D1-Region) von α-GAL codiert, umfasst eine Basensequenz, die in SEQ ID NO: 2 dargelegt ist, oder eine Basensequenz, die 95% oder mehr mit der Basensequenz identisch ist, die in SEQ ID NO: 2 dargelegt ist, und eine identische Funktion aufweist.

2. B-Zell-Antikörper-Rezeptor nach Anspruch 1, wobei ein Linker in einen Bindungsteil von a), b), c) oder d) eingefügt wird.

3. B-Zell-Antikörper-Rezeptor nach Anspruch 1 oder 2, wobei die Transmembrandomäne b) ausgewählt ist aus einer Gruppe bestehend aus CD28, CD3ζ, CD4 und CD8α.

4. B-Zell-Antikörper-Rezeptor nach einem der Ansprüche 1 bis 3, wobei die intrazelluläre Domäne eines kostimulatorischen Faktors c) ausgewählt ist aus der Gruppe bestehend aus OX40, 4-1BB, CD27, CD278 und CD28.

5. B-Zell-Antikörper-Rezeptor nach einem der Ansprüche 1 bis 4, wobei der aktivierte Rezeptor d) ausgewählt ist aus einer Gruppe bestehend aus TCR und NKG2D.

6. Nukleinsäure, die den B-Zell-Antikörper-Rezeptor codiert, nach einem der Ansprüche 1 bis 5.

7. Vektor, der die Nukleinsäure nach Anspruch 6 umfasst.

8. Immunzelle, die einen B-Zell-Antikörper-Rezeptor exprimiert, der unter Verwendung der Nukleinsäure nach Anspruch 6 oder des Vektors nach Anspruch 7 transfiziert wurde.

9. Immunzelle nach Anspruch 8, wobei die Immunzelle eine αβT-Zelle, eine CD8T-Zelle, eine CD4T-Zelle, eine γδT-Zelle oder eine NK-Zelle ist.

10. Regeneratives medizinisches Produkt, umfassend die Immunzelle nach Anspruch 8 oder 9 als Wirkstoff.

11. Immunzelle, die einen B-Zell-Antikörper-Rezeptor exprimiert, nach einem der Ansprüche 1 bis 5 für die Verwendung in einem Verfahren zur Behandlung und/oder Prävention einer lysosomalen Speicherkrankheit.

12. Immunzelle für die Verwendung nach Anspruch 11, wobei die lysosomale Speicherkrankheit Morbus Fabry ist.

## Revendications

1. Récepteur d'anticorps de lymphocyte B dans lequel un domaine de liaison à un anticorps a), un domaine transmembranaire b), un domaine intracellulaire d'un facteur co-stimulateur c), et un domaine intracellulaire d'un récepteur activé d) sont combinés, dans lequel le domaine de liaison à un anticorps a) comprend :
- une protéine de pleine longueur d'α-GAL, et la séquence de bases codant pour la protéine de pleine longueur d'α-GAL comprend une séquence de bases indiquée dans SEQ ID N° : 1, ou une séquence de bases qui présente au moins 95 % d'identité avec la séquence de bases indiquée dans SEQ ID N° : 1, présentant une fonction identique ; ou
- une région de séquence de reconnaissance d'anticorps (région D1) d'α-GAL, et la séquence de bases codant pour la région de séquence de reconnaissance d'anticorps (région D1) d'α-GAL comprend une séquence de bases indiquée dans SEQ ID N° : 2, ou une séquence de bases qui présente au moins 95 % d'identité avec la séquence de bases indiquée dans SEQ ID N° : 2, présentant une fonction identique.

2. Récepteur d'anticorps de lymphocyte B selon la revendication 1, dans lequel un lieur est inséré dans un fragment de liaison desdits a), b), c) ou d).

3. Récepteur d'anticorps de lymphocyte B selon la revendication 1 ou 2, dans lequel le domaine transmembranaire b) est choisi parmi un groupe constitué de CD28, CD3ζ, CD4, et CD8α.

4. Récepteur d'anticorps de lymphocyte B selon l'une quelconque des revendications 1 à 3, dans lequel le domaine intracellulaire d'un facteur co-stimulateur c) est choisi parmi un groupe constitué de OX40, 4-1BB, CD27, CD278, et CD28.

5. Récepteur d'anticorps de lymphocyte B selon l'une quelconque des revendications 1 à 4, dans lequel le récepteur activé d) est choisi parmi un groupe constitué de TCR et NKG2D.

6. Acide nucléique codant pour le récepteur d'anticorps de lymphocyte B selon l'une quelconque des revendications 1 à 5.

7. Vecteur comprenant l'acide nucléique selon la revendication 6.

8. Cellule immunitaire exprimant un récepteur d'anticorps de lymphocyte B qui a été transfectée à l'aide de l'acide nucléique selon la revendication 6 ou du vecteur selon la revendication 7.

9. Cellule immunitaire selon la revendication 8, dans laquelle la cellule immunitaire est une cellule αβT, une cellule CD8T, une cellule CD4T, une cellule γδT, ou une cellule NK.

10. Produit médicinal régénératif comprenant la cellule immunitaire selon la revendication 8 ou 9 en tant que principe actif.

11. Cellule immunitaire exprimant un récepteur d'anticorps de lymphocyte B selon l'une quelconque des revendications 1 à 5 destinée à être utilisée dans un procédé de traitement et/ou de prévention d'une maladie de surcharge lysosomale.

12. Cellule immunitaire destinée à être utilisée selon la revendication 11, dans laquelle la maladie de surcharge lysosomale est la maladie de Fabry.
